# EUROPEAN PATENT APPLICATION

(11) **EP 4 487 855 A1**
(43) Date of publication of application: **08.01.2025**
(21) Application number: 22928881.6
(22) Date of filing: 10.11.2022
(51) Int. Cl.: A61K 33/30, A61K 9/107, A61K 47/02, A61K 47/06, A61K 47/10, A61P 17/02, A61P 35/00

(54) **OIL-IN-WATER EMULSION COMPOSITION**

(30) Priority: 28.02.2022 JP 2022029871
(71) Applicant: Otsuka Pharmaceutical Factory, Inc., Naruto-shi, Tokushima 772-8601 (JP)
(72) Inventor: SHIRAI, Katsunori, Naruto-shi, Tokushima 772-8601 (JP); NAGIRI, Toshiya, Naruto-shi, Tokushima 772-8601 (JP); TAKENAKA, Ryoichi, Naruto-shi, Tokushima 772-8601 (JP); MARUYAMA, Shiori, Naruto-shi, Tokushima 772-8601 (JP); TERAO, Toshimitsu, Naruto-shi, Tokushima 772-8601 (JP)
(74) Representative: Hoffmann Eitle
(86) International application number: PCT/JP2022/041860
(87) International publication number: WO 2023/162363

(57) **Abstract**

The purpose of the present invention is to provide a formulation which is for producing an oil-in-water emulsion composition that has zinc chloride as an active component and which provides excellent separation inhibiting properties and/or drug release properties. Provided is an oil-in-water emulsion composition comprising: (A) not less than 30 wt% of zinc chloride; (B) an oily base selected from the group consisting of hydrocarbon oils and higher alcohols; (C) a non-ionic surfactant; and (D) an aqueous base selected from the group consisting of water and polyalcohols. The content of (B) with respect to 100 parts by weight of the component (B) and the component (C) combined is not less than 52 parts by weight. The content of (B) with respect to 100 parts by weight of the component (B) and the component (D) combined is not less than 11.7 parts by weight. Thus, the oil-in-water emulsion composition has excellent separation inhibiting properties. When the component (D) is water and polyethylene glycol, the oil-in-water emulsion composition has excellent drug release properties.

## Description

### Technical Field

The present invention relates to an oil-in-water emulsion composition excellent in separation inhibiting properties and/or drug release properties.

### Background Art

Some topical preparations include drugs used for treatment of skin malignancies, skin lesions of breast cancer and the like, and their effectiveness and necessity are widely recognized. For example, a topical preparation containing zinc chloride as an active component and having an effect of stopping bleeding or exudate at a cancerous skin ulcer site that cannot be controlled by a usual hemostatic agent is known as Mohs' ointment.

This preparation has been improved from the original formulation studied by Frederic Edward Mohs, in terms of raw material availability, applicability to affected areas, and prevention of viscosity increase with time. Known improved formulations include so-called starch formulations (NPL 1), sorbitol formulations (NPL 2), macrogol formulations (NPL 3), and the like. The starch formulation is composed of readily available raw materials such as zinc chloride, zinc oxide, glycerin, water, and starch, and is actually used as an in-hospital preparation. The sorbitol formulation has improved applicability by further adding sorbitol to the starch formulation. The macrogol formulation is one that replaces the starch and sorbitol of the sorbitol formulation with macrogol and crystalline cellulose, and viscosity increase with time is relatively suppressed.

### Citation List

### Non Patent Literature

NPL 1: Medical Journal, Vol. 41, No. 9, 2005, p. 2289-2294
NPL 2: Pharmaceutics, 75 (4), 264-270 (2015)
NPL 3: The Pharmaceutical Society of Japan, 137(4) 477-484 (2017)

### Summary of Invention

### Technical Problem

Since such a topical preparation containing zinc chloride as an active component has a strong acidity, when the applied topical preparation drips to a place other than the affected area, the risk of damaging normal skin is extremely high. In the above improved formulations, there is room for improvement in terms of adhesiveness to a wet part such as a cancerous skin ulcer site although improvement is made in terms of applicability, viscosity increase inhibiting properties, and the like.

In order to improve adhesiveness to a biological tissue, it is considered effective to further blend an oily base. In order to further blend an oily base, it is necessary to be in the form of an emulsion composition, but in conventional topical preparations containing zinc chloride as an active component, formulation characteristics when formulated as an emulsion composition have not been sufficiently studied. Therefore, when the present inventors formulated a topical preparation containing zinc chloride as an active component in the form of an oil-in-water emulsion composition, the present inventors faced a problem in that the topical preparation is extremely easily separated due to the unique characteristic of the topical preparation that the acidity of the active component is strong.

On the other hand, the formulation design for improving the efficacy of a topical preparation containing zinc chloride as an active component has not been sufficiently studied, and there is room for improvement.

In view of the above points, an object of the present invention is to provide a formulation which is for producing an oil-in-water emulsion composition that has zinc chloride as an active component and which provides excellent separation inhibiting properties and/or drug release properties.

### Solution to Problem

As a result of intensive studies, the present inventors have found that in an oil-in-water emulsion composition containing zinc chloride as an active component, excellent separation inhibiting properties are exhibited by adjusting the ratio of the oily base to be within a predetermined range. It has been found that when polyethylene glycol is blended, excellent drug release properties are exhibited. The present inventors have also found that excellent discoloration inhibiting properties are exhibited by adjusting the HLB value and/or content of a non-ionic surfactant within a predetermined range in addition to the adjustment of the ratio of an oily base or the blending of polyethylene glycol, adhesiveness is further improved in the further blending a silicic acid compound, and the drug release properties are further improved by adjusting the blending amount of a silicic acid compound to a predetermined amount. The present inventors have conducted further studies based on the findings, leading to the completion of the present invention.

That is, the present invention provides inventions of the following aspects.
Item 1. An oil-in-water emulsion composition containing:
   (A) 30 wt% or more of zinc chloride;
   (B) an oily base selected from the group consisting of a hydrocarbon oil and a higher alcohol;
   (C) a non-ionic surfactant; and
   (D) an aqueous base selected from the group consisting of water and a polyalcohol,

   wherein a content of (B) with respect to 100 parts by weight of the total amount of the component (B) and the component (C) is 52 parts by weight or more, and
   a content of (B) with respect to 100 parts by weight of the total amount of the component (B) and the component (D) is 11.7 parts by weight or more.
Item 2. An oil-in-water emulsion composition containing:
   (A) 30 wt% or more of zinc chloride;
   (B) an oily base selected from the group consisting of a hydrocarbon oil and a higher alcohol;
   (C) a non-ionic surfactant; and
   (D) an aqueous base selected from the group consisting of water and polyalcohols,
   wherein the component (D) is water and polyethylene glycol.
Item 3. The oil-in-water emulsion composition according to item 1 or 2, wherein a content of the component (D) with respect to 100 parts by weight of the total amount of the component (B), the component (C), and the component (D) is 42 parts by weight or more.
Item 4. The oil-in-water emulsion composition according to any one of items 1 to 3, wherein a content of the component (C) is 12.7 wt% or less.
Item 5. The oil-in-water emulsion composition according to any one of items 1 to 4, wherein the content of the component (C) is 1 to 7.5 wt%.
Item 6. The oil-in-water emulsion composition according to any one of items 1 to 5, wherein the component (C) is one or more kinds of non-ionic surfactants, and
   an HLB value of the component (C) represented by weighted-averaging an HLB of each non-ionic surfactant by the content is 8 to 17.
Item 7. The oil-in-water emulsion composition according to any one of items 1 to 6, wherein the component (D) contains a polyalcohol, and the polyalcohol contains solid polyalkylene glycol.
Item 8. The oil-in-water emulsion composition according to any one of items 1 to 9, wherein the component (B) contains both a hydrocarbon oil and a higher alcohol.
Item 9. The oil-in-water emulsion composition according to any one of items 1 to 8, wherein the higher alcohol is a linear alcohol.
Item 10. The oil-in-water emulsion composition according to any one of items 1 to 11, further containing a silicic acid compound.
Item 11. The oil-in-water emulsion composition according to item 10, wherein a content of the silicic acid compound is 0.7 wt% or less.
Item 12. The oil-in-water emulsion composition according to any one of items 1 to 11, wherein the component (C) is selected from the group consisting of polyoxyalkylene alkyl ether and polyoxyalkylene arachyl ether.
Item 13. The oil-in-water emulsion composition according to item 12, wherein the component (C) is two or more polyoxyalkylene alkyl ethers, and hydrophobic alkyl groups of the polyoxyalkylene alkyl ethers are the same as each other.
Item 14. The oil-in-water emulsion composition according to any one of items 2 to 13, wherein the content of (B) with respect to 100 parts by weight of the total amount of the component (B) and the component (C) is 52 parts by weight or more, and
   the content of (B) with respect to 100 parts by weight of the total amount of the component (B) and the component (D) is 11.7 parts by weight or more.
Item 15. The oil-in-water emulsion composition according to any one of items 1 to 14, which is used as an inhibitor of bleeding or exudate at a cancerous skin ulcer site or as a reducing agent for a cancerous skin ulcer.
Item 16. Use of the oil-in-water emulsion composition according to any one of items 1 to 14 for suppressing bleeding or exudate at a cancerous skin ulcer site or reducing a cancerous skin ulcer.
Item 17. A method for suppressing bleeding or exudate at a cancerous skin ulcer site or a method for reducing a cancerous skin ulcer, the method including a step of applying the oil-in-water emulsion composition according to any one of items 1 to 14 to a cancerous skin ulcer patient.

### Advantageous Effects of Invention

According to the present invention, there is provided a formulation which is for producing an oil-in-water emulsion composition that has zinc chloride as an active component and which provides excellent separation inhibiting properties and/or drug release properties.

### Description of Embodiments

A first oil-in-water emulsion composition of the present invention contains (A) 30 to 60 wt% of zinc chloride (also referred to as "component (A)" hereinafter), (B) an oily base selected from the group consisting of a hydrocarbon and a higher alcohol (also referred to as "component (B)" hereinafter), (C) a non-ionic surfactant (also referred to as "component (C)" hereinafter), and (D) an aqueous base selected from the group consisting of water and a polyalcohol (also referred to as "component (D)" hereinafter), in which a content (also referred to as "ratio 1" hereinafter) of (B) with respect to 100 parts by weight of the total amount of the component (B) and the component (C) is 52 parts by weight or more, and a content (also referred to as "ratio 2" hereinafter) of (B) with respect to 100 parts by weight of the total amount of the component (B) and the component (D) is 11.7 parts by weight or more.

A second oil-in-water emulsion composition of the present invention contains (A) 30 to 60 wt% of zinc chloride (also referred to as "component (A)" hereinafter), (B) an oily base selected from the group consisting of a hydrocarbon and a higher alcohol (also referred to as "component (B)" hereinafter), (C) a non-ionic surfactant (also referred to as "component (C)" hereinafter), and (D) an aqueous base selected from the group consisting of water and a polyalcohol (also referred to as "component (D)" hereinafter), in which the component (D) is water and polyethylene glycol.

The first oil-in-water emulsion composition and the second oil-in-water emulsion composition are collectively referred to as "oil-in-water emulsion composition". Hereinafter, the oil-in-water emulsion composition of the present invention will be specifically described.

### (A) Zinc chloride

The oil-in-water emulsion composition of the present invention contains a specific concentration of zinc chloride as the component (A). Zinc chloride is a component known as a component of Mohs' ointment.

Zinc chloride has astringent and corrosive actions of a tissue based on a protein denaturing action, and a bactericidal action. Therefore, zinc chloride works well in fixing tumors, stopping bleeding and suppressing exudates associated therewith, reducing foul smell due to secondary infection, and the like. The zinc chloride used in the present invention can be used without particular limitation as long as it can be used as a topical composition. Examples thereof include zinc chloride listed in the 17th revised Japanese Pharmacopoeia.

The content of zinc chloride in the oil-in-water emulsion composition of the present invention is 30 wt% or more. The lower limit of the content of zinc chloride is preferably 40 wt% or more and more preferably 43 wt% or more. The upper limit of the content of zinc chloride is not particularly limited, and is, for example, 60 wt% or less, preferably 55 wt% or less, more preferably 50 wt% or less, and further preferably 46 wt% or less from the viewpoint of more preferably obtaining separation inhibiting properties, and the like.

### (B) Oily base

The oil-in-water emulsion composition of the present invention contains, as the component (B), a specific oily base selected from the group consisting of a hydrocarbon oil and a higher alcohol at a predetermined ratio.

The hydrocarbon oil used in the present invention may be any of a solid hydrocarbon oil, a semi-solid (paste) hydrocarbon oil, and a liquid hydrocarbon oil as long as it is pharmaceutically acceptable. Examples of the solid hydrocarbon oil include paraffin wax (solid paraffin), ceresin, petrolatum, and microcrystalline wax. Examples of the semi-solid (paste) hydrocarbon oil include petrolatum and gelled hydrocarbon. Examples of the liquid hydrocarbon oil include liquid paraffin, squalene, and squalane.

These polyvalent hydrogen oils may be used singly or in combination of a plurality of kinds thereof.

Among these hydrocarbons, a solid hydrocarbon oil is preferred, and paraffin wax (solid paraffin) is more preferred.

The higher alcohol may be a pharmaceutically acceptable monohydric alcohol having 6 or more carbon atoms, and is, for example, a monohydric alcohol having 8 to 24 carbon atoms, preferably a monohydric alcohol having 10 to 22 carbon atoms, more preferably a monohydric alcohol having 14 to 20 carbon atoms, and further preferably a monohydric alcohol having 16 to 18 carbon atoms.

The carbon chain of the higher alcohol used in the present invention may be either linear or branched. The carbon chain of the higher alcohol used in the present invention may be either a saturated type or an unsaturated type, and a saturated type is preferred.

Specific examples of the higher alcohol used in the present invention include linear higher alcohols such as caprylic alcohol, lauryl alcohol, myristyl alcohol, cetanol, stearyl alcohol, oleyl alcohol, linoleyl alcohol, arachidyl alcohol, behenyl alcohol, and linoglyceryl alcohol; and branched higher alcohols such as isostearyl alcohol and 2-octyldodecanol. These higher alcohols may be used singly or in combination of two or more kinds thereof.

From the viewpoint of further improving the separation inhibiting properties, the higher alcohol used in the present invention is preferably a linear higher alcohol (that is, not including a branched alcohol as a higher alcohol), more preferably a linear monohydric alcohol having 10 to 22 carbon atoms, further preferably a linear monohydric alcohol having 14 to 20 carbon atoms, still more preferably a linear monohydric alcohol having 16 to 18 carbon atoms, and particularly preferably cetanol and stearyl alcohol.

In the present invention, as the component (B), either or both of a hydrocarbon oil and a higher alcohol may be used, but from the viewpoint of improving the separation inhibiting properties and/or the discoloration inhibiting properties, both a hydrocarbon oil and a higher alcohol are preferably used.

In the first oil-in-water emulsion composition of the present invention, the content (ratio 1) of (B) with respect to 100 parts by weight of the total amount of the component (B) and the component (C) is 52 parts by weight or more. In the second oil-in-water emulsion composition of the present invention, the content (ratio 1) of (B) with respect to 100 parts by weight of the total amount of the component (B) and the component (C) is preferably 52 parts by weight or more. From the viewpoint of further improving the separation inhibiting properties, the ratio 1 is preferably 60 parts by weight or more, more preferably 65 parts by weight or more, and further preferably 70 parts by weight or more. The upper limit of the ratio 1 is not particularly limited, and is, for example, less than 100 parts by weight, preferably 95 parts by weight or less, more preferably 90 parts by weight or less, and further preferably 80 parts by weight or less.

In the first oil-in-water emulsion composition of the present invention, the content (ratio 2) of (B) with respect to 100 parts by weight of the total amount of the component (B) and the component (D) is 11.7 parts by weight or more. In the second oil-in-water emulsion composition of the present invention, the content (ratio 2) of (B) with respect to 100 parts by weight of the total amount of the component (B) and the component (D) is preferably 11.7 parts by weight or more. From the viewpoint of further improving the separation inhibiting properties, the ratio 2 is preferably 15.5 parts by weight or more, more preferably 25 parts by weight or more, and further preferably 30 parts by weight or more. The upper limit of the ratio 2 is not particularly limited, and is, for example, 63 parts by weight or less, preferably 50 parts by weight or less, more preferably 45 parts by weight or less, further preferably 40 parts by weight or less, and still more preferably 35 parts by weight or less.

The specific content (total amount) of the component (B) in the oil-in-water emulsion composition of the present invention is, for example, 6 to 46.5 wt%, preferably 11.5 to 46.5 wt%, further preferably 13 to 35 wt%, and preferably 15 to 32 wt%.

In the oil-in-water emulsion composition of the present invention, the content of the component (B) with respect to 1 part by weight of the component (A) is preferably 0.34 to 0.73 parts by weight.

When the component (B) in the oil-in-water emulsion composition of the present invention contains a hydrocarbon oil, the specific content of the hydrocarbon oil is, for example, 0.8 to 16 wt% and preferably 9 to 14 wt%.

When the component (B) in the oil-in-water emulsion composition of the present invention contains a higher alcohol, the specific content of the higher alcohol is, for example, 3 to 16 wt% and preferably 4 to 8 wt%.

When the component (B) in the oil-in-water emulsion composition of the present invention contains a hydrocarbon oil and a higher alcohol, the content of the higher alcohol per 1 part by weight of the hydrocarbon oil is, for example, 0.2 to 8.2 parts by weight and preferably 0.35 to 1 part by weight.

### (C) Non-ionic surfactant

The oil-in-water emulsion composition of the present invention contains a non-ionic surfactant as the component (C).

Examples of the non-ionic surfactant used in the present invention include polyoxyalkylene alkyl ether, polyoxyalkylene arachyl ether, polymers of ethylene oxide and/or propylene oxide, polyoxyethylene-hydrogenated castor oil, glycerin fatty acid ester, polyglycerin fatty acid ester, sorbitan fatty acid ester, propylene glycol fatty acid ester, polyoxyethylene glycerin fatty acid ester, and polyoxyethylene sorbitol fatty acid ester. These non-ionic surfactants may be used singly or in combination of a plurality of kinds thereof.

Among these non-ionic surfactants, from the viewpoint of further improving the separation inhibiting properties and/or from the viewpoint of improving the discoloration inhibiting properties, polyoxyalkylene alkyl ether and polyoxyalkylene arachyl ether are preferred.

The polyoxyalkylene alkyl ether is an ether of polyalkylene glycol and a higher alcohol. Examples of the higher alcohol include monohydric alcohols having 8 to 24 carbon atoms, and specific examples thereof include linear higher alcohols such as caprylic alcohol, lauryl alcohol, myristyl alcohol, cetanol, stearyl alcohol, oleyl alcohol, linoleyl alcohol, arachidyl alcohol, behenyl alcohol, and linoglyceryl alcohol, and preferably include stearyl alcohol and behenyl alcohol. Examples of the alkylene group of the polyalkylene glycol include alkylene groups having 2 to 3 carbon atoms, and specific examples of the polyalkylene glycol include polyethylene glycol, polypropylene glycol, and combinations thereof, and polyethylene glycol is preferred. The alkylene oxide addition number in the polyoxyalkylene alkyl ether is, for example, 2 to 35 and preferably 2 to 20. These polyoxyalkylene alkyl ethers may be used singly or in combination of a plurality of kinds thereof.

The polyoxyalkylene arachyl ether is an ether of polyalkylene glycol and arachyl alcohol. Examples of the arachyl alcohol include benzyl alcohol and phenethyl alcohol. Examples of the alkylene group of the polyalkylene glycol include alkylene groups having 2 to 3 carbon atoms, and specific examples of the polyalkylene glycol include polyethylene glycol, polypropylene glycol, and combinations thereof, and polyethylene glycol is preferred. The alkylene oxide addition number in the polyoxyalkylene arachyl ether is, for example, 2 to 35 and preferably 2 to 20. These polyoxyalkylene arachyl ethers may be used singly or in combination of a plurality of kinds thereof.

In the present invention, either one of polyoxyalkylene alkyl ether and polyoxyalkylene arachyl ether may be used, or both of them may be used in combination, but from the viewpoint of further improving the separation inhibiting properties, polyoxyalkylene alkyl ether is preferably used.

In the present invention, in the case of using a plurality of (that is, two or more) of polyoxyalkylene alkyl ethers, the hydrophobic alkyl groups (that is, the alkyl groups derived from the higher alcohol described above) of the respective polyoxyalkylene alkyl ethers may be the same or different, but are preferably the same as each other from the viewpoint of further improving the separation inhibiting properties.

The HLB value of each non-ionic surfactant used as the component (C) is not particularly limited, and is, for example, 7 to 19. The HLB value represented by weighted-averaging an HLB of each of one or more kinds of non-ionic surfactants used as the component (C) by the content (also referred to as "HLB value of the component (C)" hereinafter) is not particularly limited, and is, for example, 8 to 17. From the viewpoint of improving the discoloration inhibiting properties, the HLB value of the component (C) is preferably 11.5 to 15, 11.5 to 13, or 11.5 to 12. As for the method for calculating the HLB value of the component (C), for example, when 3 wt% of a non-ionic surfactant having an HLB of 8 and 2 wt% of a non-ionic surfactant having an HLB of 17 are used in combination as the component (C), each HLB is weighted and averaged by weighting based on the content to calculate the HLB value as {8 × 3/(3 + 2)} + {17 × 2/(3 + 2)} = 11.6.

The amount of the component (C) in the oil-in-water emulsion composition of the present invention is not particularly limited as long as the relationship with the amount of the component (B) is as described above (that is, the content of (B) with respect to 100 parts by weight of the total amount of the component (B) and the component (C) is 52 parts by weight or more). The specific content of the component (C) in the oil-in-water emulsion composition of the present invention is, for example, 12.7 wt% or less. From the viewpoint of improving the discoloration inhibiting properties, the specific content of the component (C) is preferably 1 to 7.5 wt%, and more preferably 1.5 to 7.5 wt%, 3 to 7 wt%, or 4 to 6 wt%.

In the oil-in-water emulsion composition of the present invention, the content of the component (C) with respect to 1 part by weight of the component (A) is preferably 0.09 to 0.14 parts by weight.

### (D) Aqueous base

The oil-in-water emulsion composition of the present invention contains an aqueous base selected from the group consisting of water and a polyalcohol as the component (D). The first oil-in-water emulsion composition of the present invention usually contains water and preferably contains both water and a polyalcohol as the component (D). In the second oil-in-water emulsion composition of the present invention, the component (D) is water and polyethylene glycol.

The polyalcohol used in the first oil-in-water emulsion composition of the present invention is not particularly limited as long as it is pharmaceutically acceptable. Specific examples of the polyalcohol used in the present invention include dihydric alcohols such as propylene glycol, 1,3-butylene glycol, ethylene glycol, isoprene glycol, diethylene glycol, dipropylene glycol, and polyethylene glycol; and trihydric alcohols such as glycerin.

In the first oil-in-water emulsion composition and the second oil-in-water emulsion composition of the present invention, the molecular weight of polyethylene glycol is not particularly limited, and is, for example, 3600 to 22000 in terms of number average molecular weight calculated based on a hydroxyl value measured in accordance with JIS K 1557. That is, the polyethylene glycol may be any of polyethylene glycol that is liquid at 25°C, polyethylene glycol that is semi-solid (paste) at 25°C, and polyethylene glycol that is solid at 25°C. These polyalcohols may be used singly or in combination of a plurality of kinds thereof. Specific examples of the polyethylene glycol include Macrogol 400 (number average molecular weight: 400), Macrogol 1500 (number average molecular weight: 550), Macrogol 4000 (number average molecular weight: 3100), Macrogol 6000 (number average molecular weight: 8600), and Macrogol 20000 (number average molecular weight: 20000) listed in the 17th revised Japanese Pharmacopoeia.

In the first oil-in-water emulsion composition of the present invention, these polyalcohols may be used singly or in combination of two or more kinds thereof.

In the first oil-in-water emulsion composition of the present invention, among these polyalcohols, dihydric alcohols is preferred. From the viewpoint of improving the discoloration inhibiting properties, the component (D) preferably contains semi-solid or solid polyethylene glycol, more preferably contains solid polyethylene glycol, more preferably contains polyethylene glycol having a number average molecular weight of 2500 to 10000, and further preferably contains polyethylene glycol having a number average molecular weight of 2800 to 4500. In the second oil-in-water emulsion composition of the present invention, from the viewpoint of further improving the drug release properties, the polyethylene glycol preferably contains solid polyethylene glycol, more preferably contains polyethylene glycol having a number average molecular weight of 2500 to 10000, and further preferably contains polyethylene glycol having a number average molecular weight of 2800 to 4500.

The amount of the component (D) in the first oil-in-water emulsion composition of the present invention is not particularly limited as long as the relationship with the amount of the component (B) is as described above (that is, the content of (B) with respect to 100 parts by weight of the total amount of the component (B) and the component (D) is 11.7 parts by weight or more). The amount of the component (D) in the second oil-in-water emulsion composition of the present invention is preferably set to such an amount that the relationship with the amount of the component (B) is as described above (that is, the content of (B) with respect to 100 parts by weight of the total amount of the component (B) and the component (D) is 11.7 parts by weight or more). The specific content (total amount) of the component (D) in the oil-in-water emulsion composition of the present invention is, for example, 16 to 50 wt%, preferably 25 to 40 wt%, and further preferably 30 to 36 wt%.

In the oil-in-water emulsion composition of the present invention, the content of the component (D) with respect to 1 part by weight of the component (A) is preferably 0.56 to 0.91 parts by weight.

From viewpoint of improving the applicability of the oil-in-water emulsion composition, the amount of the component (D) in the oil-in-water emulsion composition of the present invention is preferably 42 parts by weight or more, 48 parts by weight or more, or 52 parts by weight or more in terms of the content (also referred to as "ratio 3" hereinafter) of the component (D) with respect to 100 parts by weight of the total amount of the component (B), the component (C), and the component (D). The upper limit of the ratio 3 is not particularly limited, and is, for example, 76 parts by weight or less, 70 parts by weight or less, or 60 parts by weight or less.

The content of water in the oil-in-water emulsion composition of the present invention is, for example, 10 to 50 wt%, preferably 14 to 42 wt%, more preferably 20 to 34 wt%, and further preferably 25 to 30 wt%.

When the first oil-in-water emulsion composition of the present invention contains a polyalcohol, the content of the polyalcohol is, for example, 0.5 to 22 wt%, preferably 1 to 20 wt%, more preferably 2.5 to 10 wt%, and further preferably 3 to 7 wt%. The content of polyethylene glycol contained in the second oil-in-water emulsion composition of the present invention is, for example, 0.5 to 22 wt%, preferably 1 to 20 wt%, more preferably 2.5 to 10 wt%, and further preferably 3 to 7 wt%.

When the component (D) in the first oil-in-water emulsion composition of the present invention contains water and a polyalcohol, the content of the polyalcohol per 1 part by weight of water is, for example, 0.02 to 0.85 parts by weight, preferably 0.05 to 0.5 parts by weight, and more preferably 0.1 to 0.3 parts by weight. The content of the polyethylene glycol per 1 part by weight of water in the second oil-in-water emulsion composition of the present invention is, for example, 0.02 to 0.85 parts by weight, preferably 0.05 to 0.5 parts by weight, and more preferably 0.1 to 0.3 parts by weight.

### Silicic acid compound

The oil-in-water emulsion composition of the present invention can further contain a silicic acid compound. The silicic acid compound can be blended for the purpose of improving the adhesiveness and/or drug release properties of the oil-in-water emulsion composition.

The silicic acid compound is a compound containing silicon dioxide (SiO₂) as a constituent element, and specific examples thereof include silicon dioxide, aluminum silicate, magnesium silicate, magnesium aluminum silicate, sodium magnesium silicate, and calcium silicate. These silicic acid compounds may be used singly or in combination of a plurality of kinds thereof. Among these silicic acid compounds, silicon dioxide is preferred.

When the oil-in-water emulsion composition of the present invention contains a silicic acid compound, the content of the silicic acid compound is, for example, 0.1 to 3 wt%, preferably 0.3 to 2 wt%, and more preferably 0.4 to 0.8 wt%.

When the oil-in-water emulsion composition of the present invention contains a silicic acid compound, the content of the silicic acid compound is preferably 0.7 wt% or less, more preferably 0.6 wt% or less, and further preferably 0.55 wt% or less from the viewpoint of further improving the drug release properties. The lower limit of the content of the silicic acid compound is not particularly limited, and is, for example, 0.1 wt% or more, 0.3 wt% or more, or 0.4 wt% or more.

### Other components

The oil-in-water emulsion composition of the present invention may contain, in addition to the above components, other bases and/or additives usually used for topical preparations for skin and the like as necessary. Such a base is not particularly limited as long as it is pharmaceutically acceptable, and examples thereof include an oily base other than the component (B), such as ester oil, fatty acid triglyceride, higher fatty acid, and silicone oil; an aqueous base other than the component (D), such as a monovalent lower (1 to 5 carbon atoms) alcohol; and a surfactant other than the component (C), such as an anionic surfactant, a cationic surfactant, and an amphoteric surfactant. Examples of the additives include cellulose derivatives (such as methyl cellulose, ethyl cellulose, hypromellose, hydroxyethyl cellulose, hydroxypropyl cellulose, methyl hydroxyethyl cellulose, and esterified products thereof), polyvinyl alcohol, colorants (such as titanium dioxide), flavoring agents, pH adjusting agents, wetting agents (such as sodium dl-pyrrolidone carboxylate solution and D-sorbitol solution), stabilizers (such as dibutylhydroxytoluene, butylhydroxyanisole, sodium edetate, sodium metaphosphate, L-arginine, L-aspartic acid, DL-alanine, glycine, sodium erythorbate, propyl gallate, sodium sulfite, sulfur dioxide, chlorogenic acid, catechin, and rosemary extract), antioxidants, ultraviolet absorbers, chelating agents, buffers, solubilizing agents, solubilizers, and preservatives.

The oil-in-water emulsion composition of the present invention may contain, in addition to the above components, a drug used for treatment of skin malignancies, skin lesions of breast cancer and the like as necessary. Examples of such a drug include zinc oxide and zinc oxide preparations (such as zinc oxide starch, zinc oxide ointment, and phenol and zinc oxide liniment).

### Properties, preparation form, etc.

The preparation form of the oil-in-water emulsion composition of the present invention is not particularly limited, but is usually a cream from the viewpoint of applicability and retention on the skin. Specific embodiments of the topical composition of the present invention usually include topical pharmaceuticals.

The pH (25°C) of the oil-in-water emulsion composition of the present invention is, for example, 0.6 to 2.6. As the consistency of the oil-in-water emulsion composition of the present invention, the consistency at 25°C measured in accordance with JIS K 2220 is, for example, 80 to 130 (1/10 mm), preferably 85 to 125 (1/10 mm), more preferably 95 to 120 (1/10 mm), and further preferably 100 to 110 (1/10 mm).

The oil-in-water emulsion composition of the present invention may be either an agent prepared before use or a commercially available preparation, but a commercially available preparation is particularly preferred because the oil-in-water emulsion composition of the present invention is excellent in the separation inhibiting properties.

### Use application

The oil-in-water emulsion composition of the present invention is used by being applied to an affected area where bleeding or exudate is observed at a cancerous skin ulcer site. Such an affected area has an extensive cancerous skin ulcer due to advanced skin cancer or cancers other than skin cancer exposed on the body surface (for example, breast cancer, head and neck cancer, and the like), and bleeding or exudate is observed. The oil-in-water emulsion composition of the present invention can be used for the purpose of improving quality of life (QOL) against such bleeding or exudate at a cancerous skin ulcer site, or infection or foul smell caused by these. The oil-in-water emulsion composition of the present invention may be used for the purpose of reducing cancerous skin ulcers.

The oil-in-water emulsion composition of the present invention can be used, for example, in the same manner as in application of Mohs' method. Specifically, the topical composition of the present invention is applied to the surface of a cancerous skin ulcer to fix a tumor tissue. In the fixation of a tumor tissue, the tumor tissue can be degenerated and necrosed by hardening a tumor blood vessel, and the necrotic tissue can be fixed and dried. After confirming the fixation of the tumor tissue, the applied oil-in-water emulsion composition can be removed as necessary.

### Dosage and administration

The oil-in-water emulsion composition of the present invention can be used in a dosage and administration according to the size of the affected area where bleeding or exudate is observed at a cancerous skin ulcer site and the degree of bleeding or exudate, and for example, it can be used in dosage and administration based on the Mohs' method. For example, the oil-in-water emulsion composition can be applied to the affected area about once every 7 to 10 days for a period according to symptoms of the affected area.

### Production method

The oil-in-water emulsion composition of the present invention can be produced by a known method for producing an emulsion preparation. Examples of the method for producing an emulsion composition of the present invention include the following method. First, the component (B) and another oily component added as necessary are mixed to prepare an oil phase composition. Separately, the component (A), the component (D), and another water-soluble component added as necessary are mixed to prepare an aqueous phase composition. The component (C) may be blended with any one or both of the aqueous phase composition and the oil phase composition, but can be preferably blended with the oil phase composition. Next, the aqueous phase composition and the oil phase composition thus obtained are mixed and emulsified by an emulsification method such as a homogenizer, thereby obtaining the oil-in-water emulsion composition of the present invention.

### Examples

Hereinafter, the present invention will be more specifically described by means of Examples; however, the present invention is not limited thereto.

### Test Example 1

The oil-in-water emulsion compositions shown in Tables 1 to 10 were produced by the following procedure. First, the component (B) and the component (C) shown in Tables 1 to 10 were mixed and dissolved by heating to prepare an oil phase composition. Separately, the component (A), the component (D), and light anhydrous silicic acid shown in Tables 1 to 10 were mixed to prepare an aqueous phase composition. The aqueous phase composition was gradually added to the temperature-adjusted oil phase composition to refine particles (oil droplets) using a homomixer, and the mixture was cooled with stirring to obtain an oil-in-water emulsion composition. The obtained oil-in-water emulsion composition was evaluated for the following <1> Separation inhibiting properties. For some compositions, evaluations or measurements of the following <2> to <8> were performed. The results are shown in Tables 1 to 10.

### <1> Separation inhibiting properties

The separation inhibiting properties were evaluated for all the oil-in-water emulsion compositions. Specifically, 20 to 30 g of the oil-in-water emulsion composition was put in a glass bottle (inner diameter: about 30 mm) and stored at 50°C for 4 weeks. The appearance of the oil-in-water emulsion composition after storage was visually evaluated according to the following criteria.
∘: No separation was observed.
Δ: The aqueous phase was separated slightly (the aqueous phase thickness was less than 5% of the entirety).
×: The aqueous phase was separated somewhat (the aqueous phase thickness was 5% or more and less than 10% of the entirety).
××: The aqueous phase was separated considerably (the aqueous phase thickness was 10% or more of the entirety).

### <2> Lack of fluidity

The lack of fluidity was evaluated for the oil-in-water emulsion compositions of Examples 1 to 25. Specifically, about 5.5 g of the oil-in-water emulsion composition was evenly spread on a horizontal plastic plate in a circular region having an inner diameter of 30 mm and a height of 5 mm, the plastic plate was tilted by 90° from the horizontal plane, and whether or not the oil-in-water emulsion composition protruded from the circular region was evaluated within 15 minutes. A case where the oil-in-water emulsion composition did not protrude was evaluated as "o", and a case where the oil-in-water emulsion composition protruded was evaluated as "×".

### <3> Applicability

The applicability (comprehensive evaluation of softness and spreadability) was evaluated for the oil-in-water emulsion compositions of Examples 1 to 25, 57 to 66, 73, 74, 76, 81, 83 to 85, 87 to 90, 92 to 95, 97, 99, 101, 104, 105, 107, 114, and 125. Specifically, after wearing gloves, the oil-in-water emulsion composition was scooped up with the first joint part of the index finger, and applied to the chicken breast, and the applicability was evaluated according to the following criteria.
⊙: The oil-in-water emulsion composition is excellent in softness and spreadability and can be easily applied.
∘: The oil-in-water emulsion composition is excellent in softness and spreadability and thus can be applied.
Δ: The oil-in-water emulsion composition is slightly hard and poor in spreadability, so that application is difficult (application itself is possible).
×: The oil-in-water emulsion composition is hard and is difficult to spread, and cannot be applied.

### <4> Discoloration inhibiting properties

The discoloration inhibiting properties were evaluated for the oil-in-water emulsion compositions of Examples 1 and 3 to 53. Specifically, 20 to 30 g of the oil-in-water emulsion composition was put in a glass bottle (inner diameter: about 30 mm) and stored at 50°C for 4 weeks. The appearance of the oil-in-water emulsion composition after storage was visually evaluated according to the following criteria.
∘: The appearance was the same as before storage.
Δ: The appearance was slightly colored compared to before storage.
×: The appearance was apparently colored compared to before storage.

### <5> Adhesion time

The adhesion time was evaluated for the oil-in-water emulsion compositions of Examples 10, 59, 64 to 66, 73, 74, 76, 81, 83 to 90, 92, 94, 95, 97, 99, 101, 104, 105, and 107. Specifically, about 5.5 g of the oil-in-water emulsion composition was evenly spread on a horizontal chicken breast in a circular region having an inner diameter of 30 mm and a height of 0.5 mm, the chicken breast was tilted by 90° from the horizontal plane, and the time (min) taken for the oil-in-water emulsion composition to fall was measured. The measurement time was evaluated up to 60 minutes. The closer the adhesion time is to 60 minutes, the more excellent the adhesiveness can be evaluated.

### <6> pH

The pH of each of the oil-in-water emulsion compositions of Examples 10, 83, 84, 89, 90, 95, 99, 100, 103 to 105, and 107 was measured at 20 to 25°C using a pH meter.

### <7> Drug release amount

The drug release properties were evaluated for the oil-in-water emulsion compositions of Examples 10, 57 to 61, 64 to 65, 70, 71, 73 to 75, 81, 83, 85, 87 to 90, 92 to 94, 97, 101, 104, 107, 118, and 126. Specifically, a stirrer was put in a glass bottle having an inner diameter of about 30 mm and a height of 64 mm, the inside of the glass bottle was filled with about 30 g of purified water, the oil-in-water emulsion composition was evenly filled in a circular ring having an inner diameter of 15 mm and a height of 5 mm, which was placed on the upper surface of a filter having a pore diameter of 0.22 µm, and the lower surface of the filter was placed to be in contact with the water surface in the glass bottle. The rotation speed of the stirrer was set to 300 rpm, and the mixture was stirred for 15 minutes. The purified water in the glass bottle was collected, and the amount (mg) of zinc chloride released from the oil-in-water emulsion composition in the circular ring into the purified water through the filter was measured.

### <8> Consistency

For the oil-in-water emulsion compositions of Examples 10, 57 to 61, 64 to 66, 73, 74, 76, 81, 83 to 85, 87 to 90, 92 to 95, 97, 99 to 101, 103 to 105, and 107, the consistency (1/10 mm) was measured. Specifically, the oil-in-water emulsion composition was used as a sample as it was, a sample adjusted to 25°C was filled in a measurement container, and measurement was performed according to JIS K 2220 in a state where the surface of the sample was flat except for an excessive sample. For the measurement, Micro-cone, suitable container, and shaft described in EUROPEAN PHARMACOPEIA 9.0, 2.9.9 MEASUREMENT OF CONSISTENCY BY PENETROMETRY Figure 2.9.9.-3 were used.

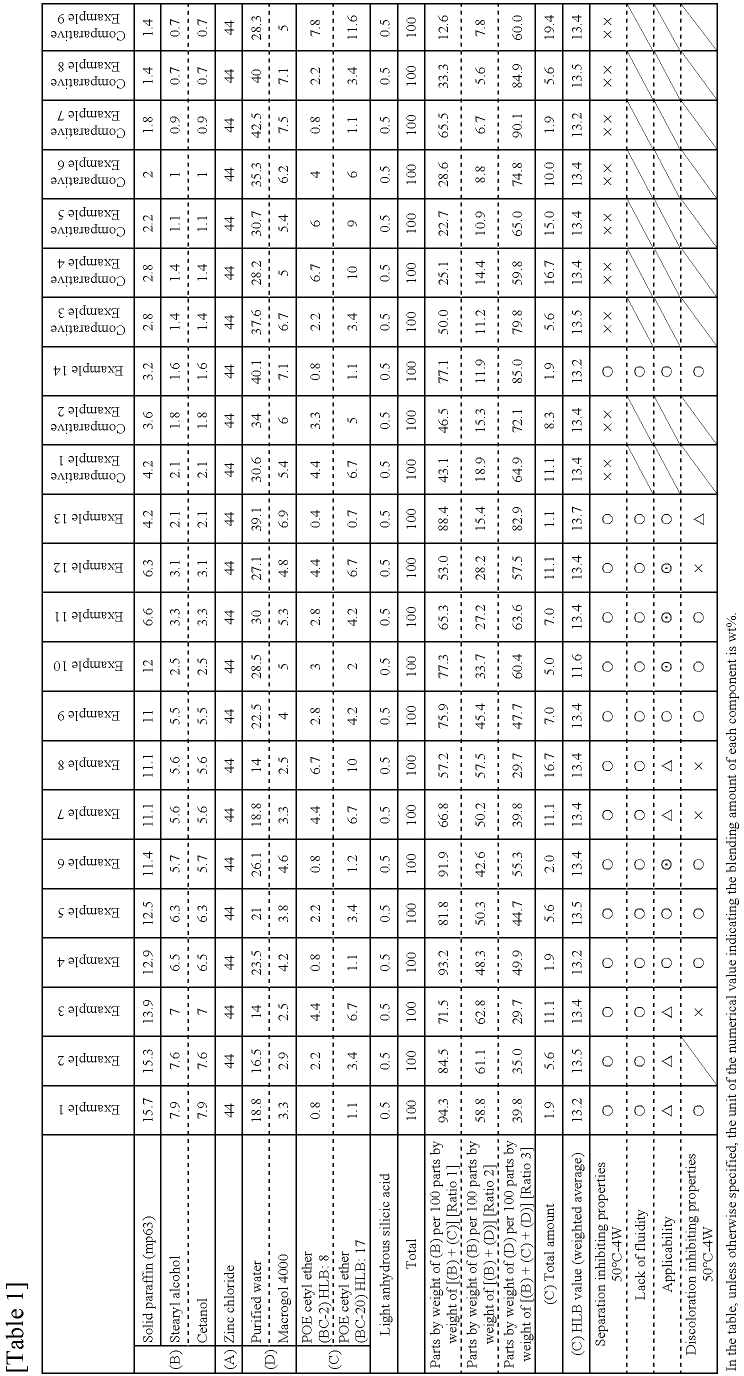

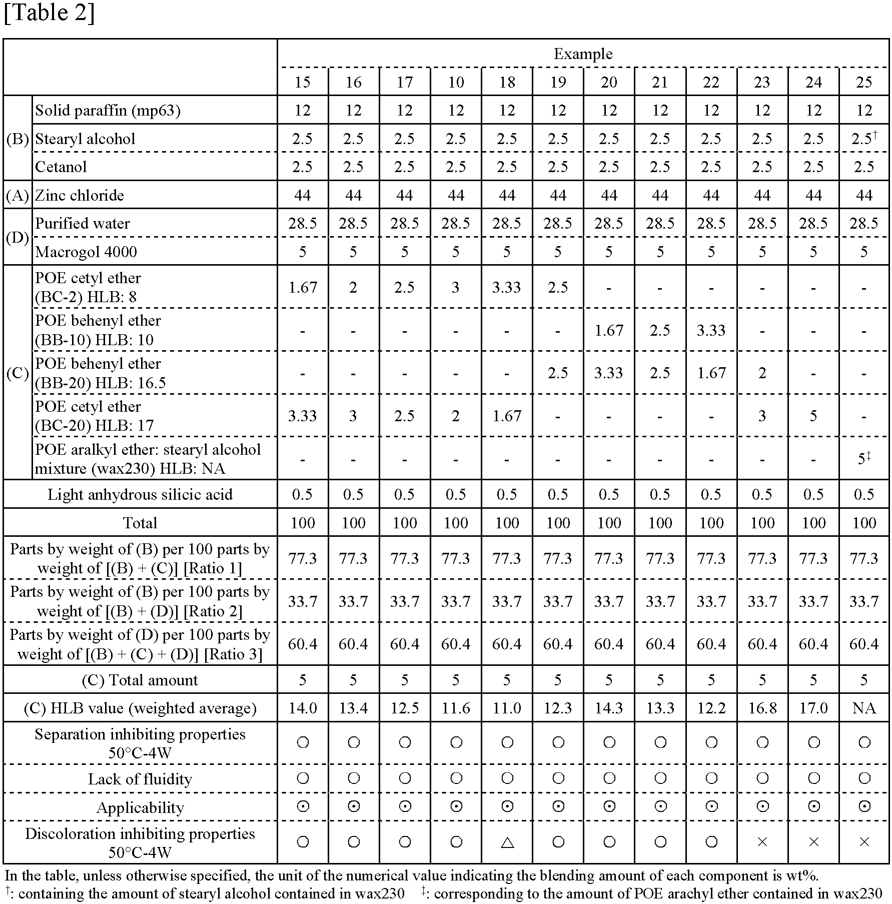

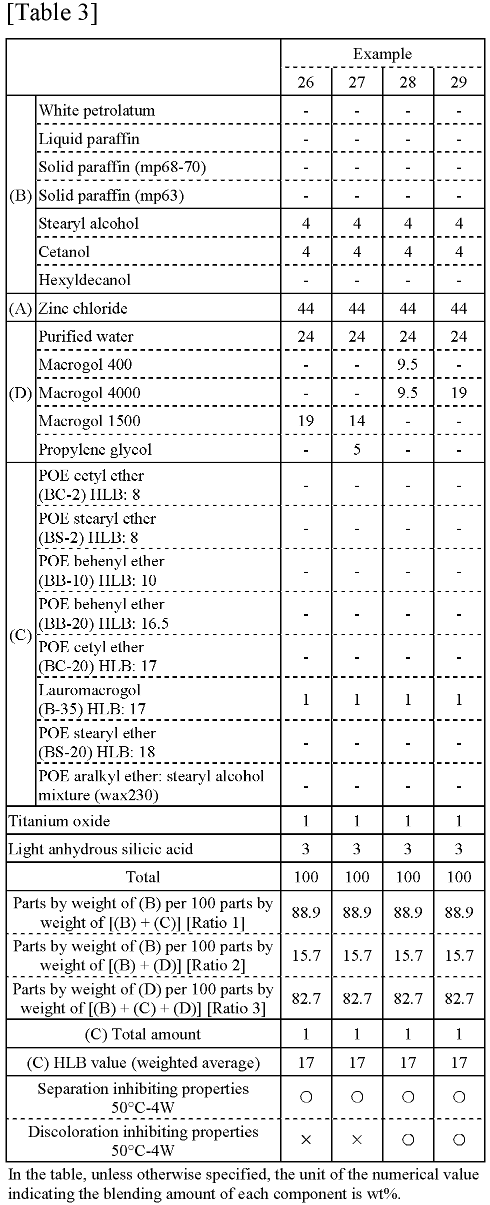

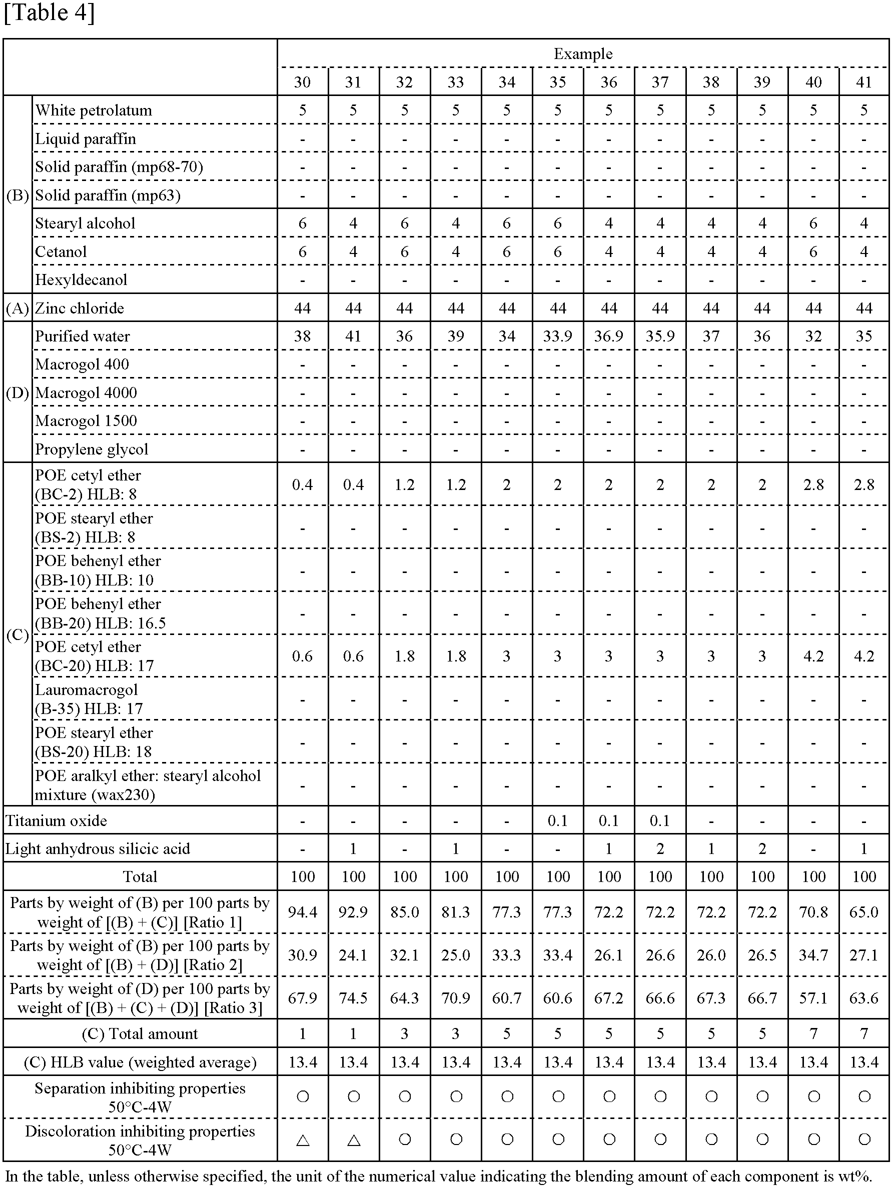

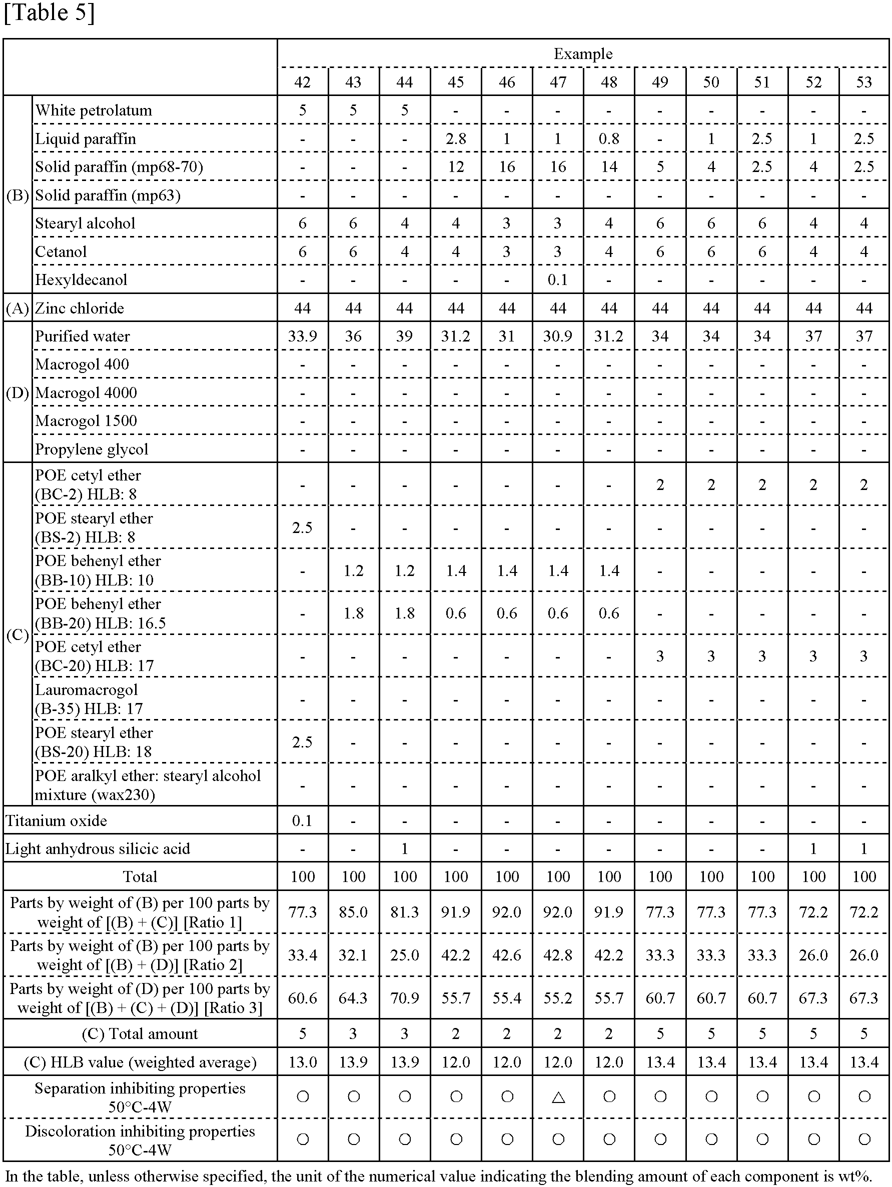

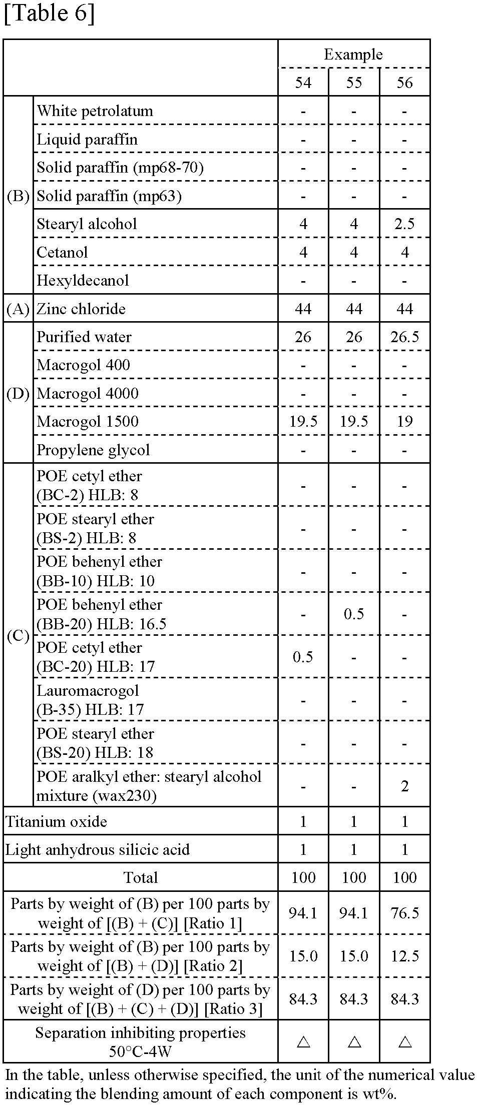

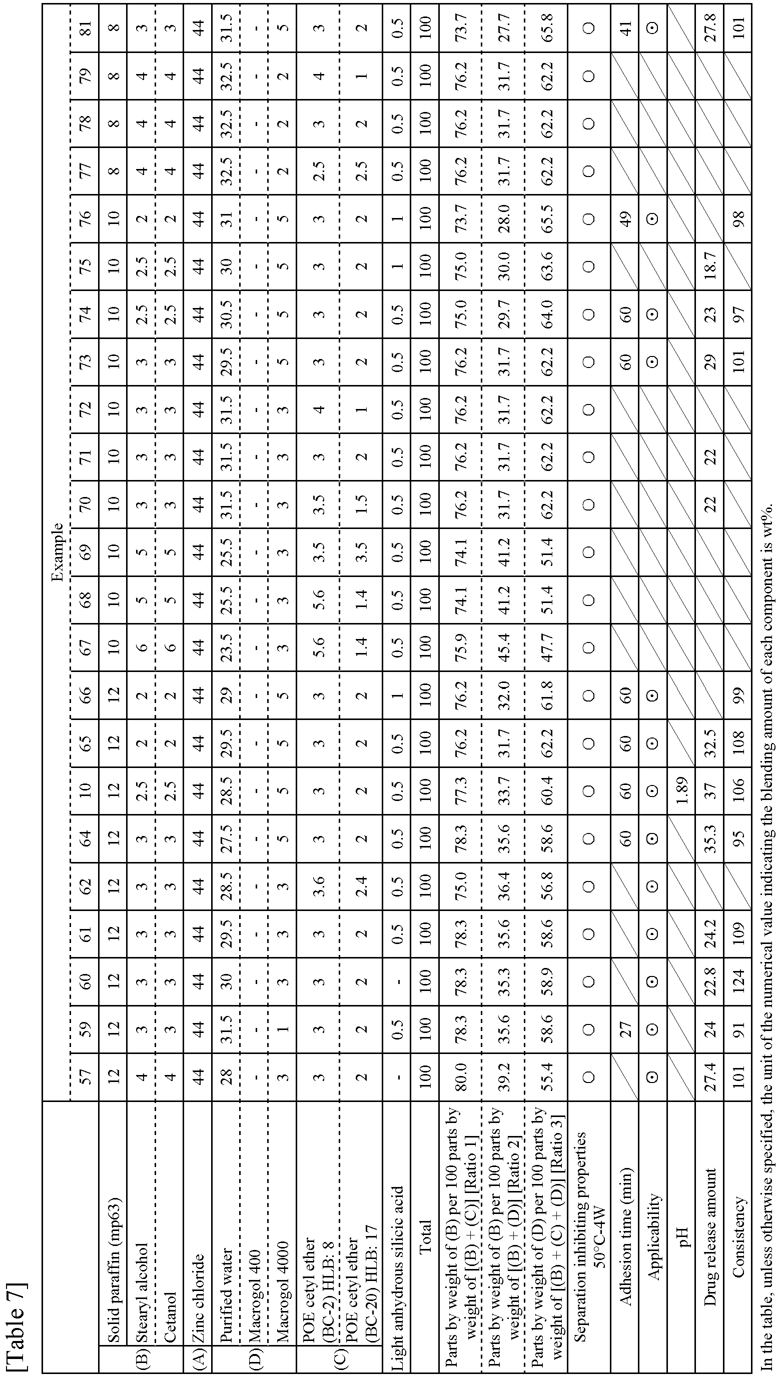

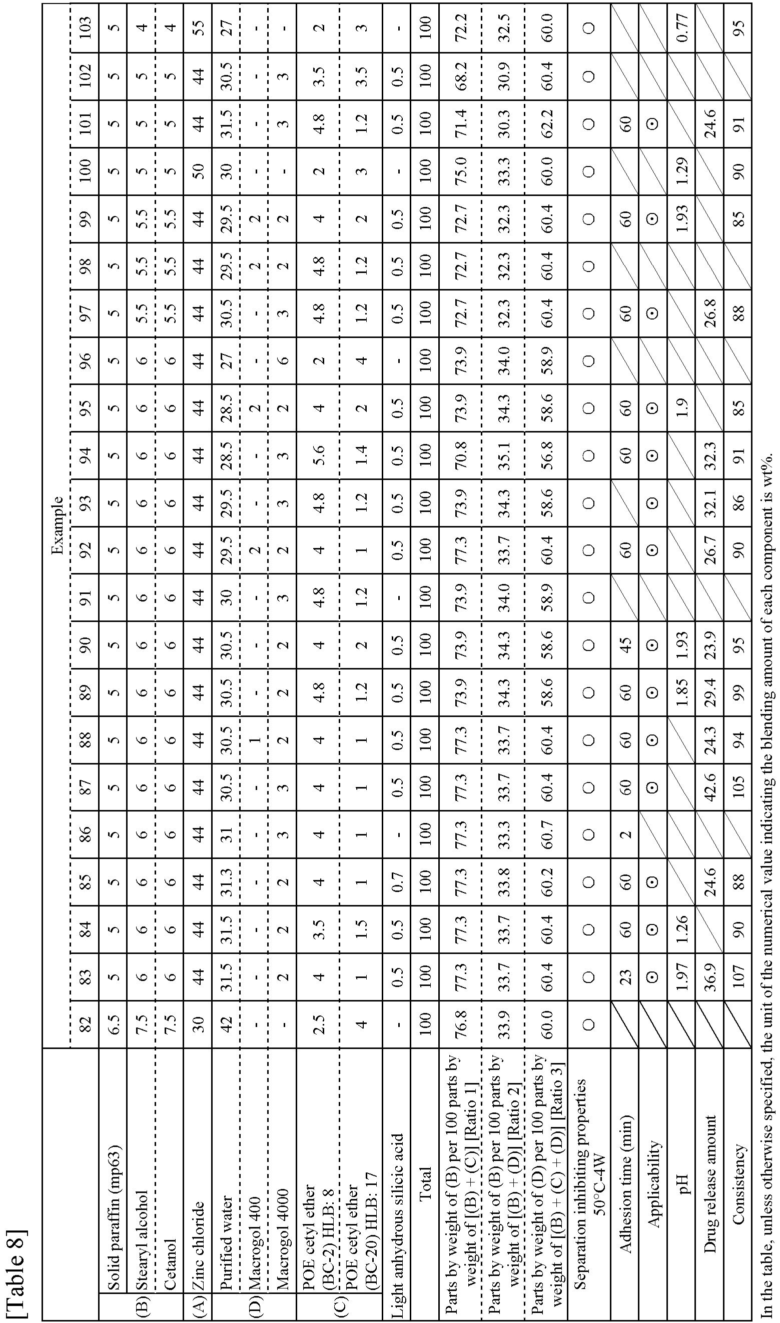

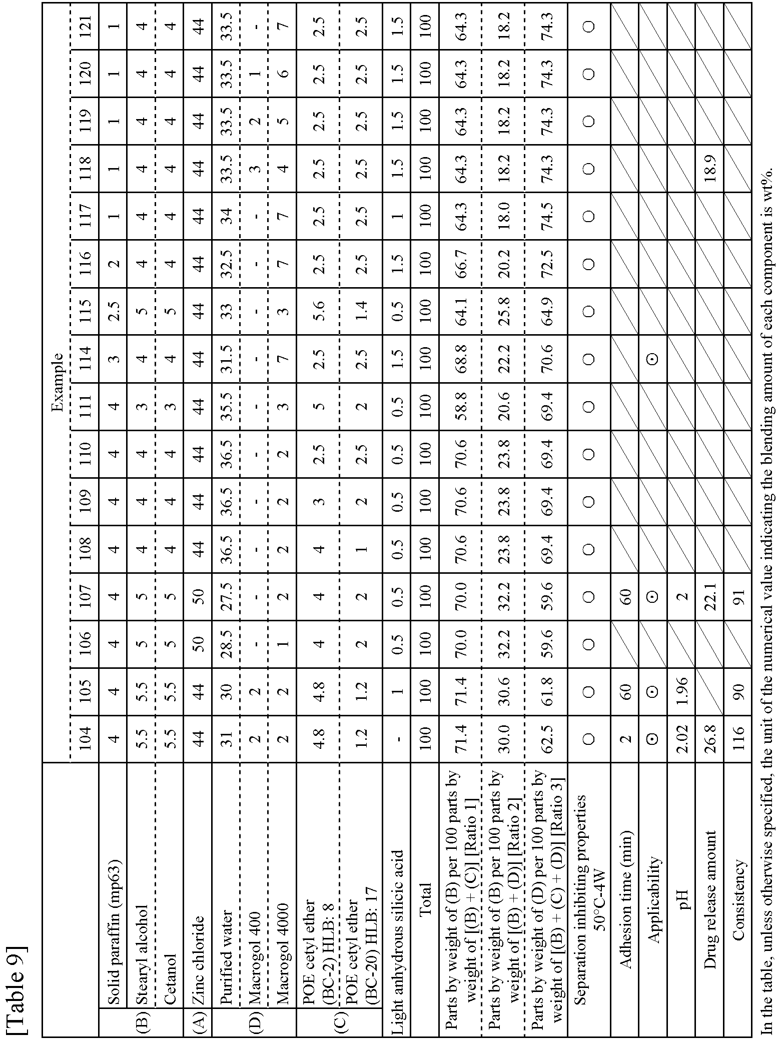

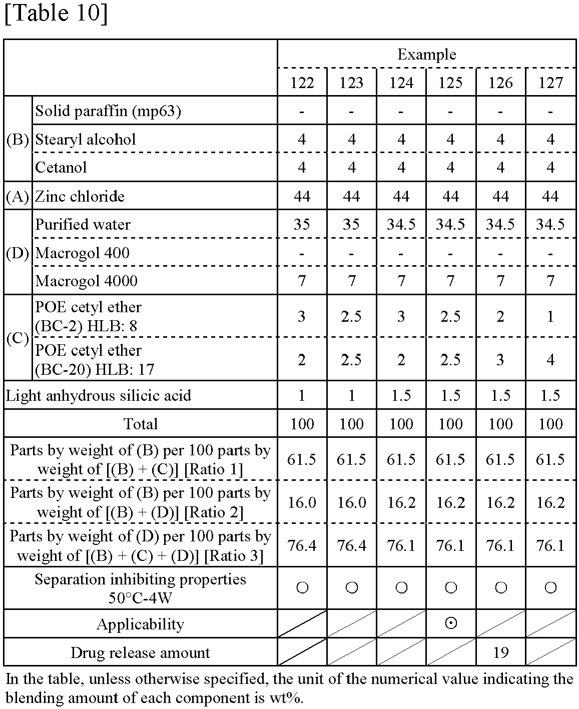

As shown in the comparison between Examples 1 to 127 and Comparative Examples 1 to 9, in the oil-in-water emulsion composition containing: (A) 30 wt% or more of zinc chloride; (B) an oily base selected from the group consisting of a hydrocarbon oil and a higher alcohol; (C) a non-ionic surfactant; and (D) an aqueous base selected from the group consisting of water and a polyalcohol, it has been confirmed that excellent separation inhibiting properties are obtained in the case of satisfying the requirement that the content (ratio 1) of (B) with respect to 100 parts by weight of the total amount of the component (B) and the component (C) is 52 parts by weight or more, and the content (ratio 2) of (B) with respect to 100 parts by weight of the total amount of the component (B) and the component (D) is 11.7 parts by weight or more (Examples 1 to 127).

In the case of not containing a hydrocarbon and containing only a higher alcohol as the component (B) (Examples 26 to 29, 54 to 56, and 122 to 127), it has also been confirmed that more excellent separation inhibiting properties are obtained in the case of the ratio 1 of 52 to 90 parts by weight and/or the ratio 2 of 15.7 parts by weight or more (Examples 26 to 29 and 122 to 127). On the other hand, it has been confirmed that the case of containing both a hydrocarbon and a higher alcohol as the component (B) (Examples 1 to 25, 30 to 53, and 57 to 121) is further preferable in that excellent separation inhibiting properties are obtained in a wider range of 52 parts by weight or more of the ratio 1 and 11.7 parts by weight or more of the ratio 2.

As shown in the comparison between Example 46 and Example 47, it has been confirmed that the separation inhibiting properties are improved when the higher alcohol contained in the component (B) is a linear alcohol and does not contain a branched alcohol (Example 46).

Among the oil-in-water emulsion compositions of Examples 1 to 25, 57 to 66, 73, 74, 76, 81, 83 to 85, 87 to 90, 92 to 95, 97, 99, 101, 104, 105, 107, 114, and 125 tested for the applicability, when the content (ratio 3) of the component (D) with respect to 100 parts by weight of the total amount of the component (B), the component (C), and the component (D) is 42 parts by weight or more (Examples 4 to 6, 9 to 25, 57 to 66, 73, 74, 76, 81, 83 to 85, 87 to 90, 92 to 95, 97, 99, 101, 104, 105, 107, 114, and 125), the applicability was further improved.

Among the oil-in-water emulsion compositions of Examples 1 and 3 to 53 tested for the discoloration inhibiting properties, in the case of containing both a hydrocarbon oil and a higher alcohol as the component (B) (Examples 1 and 3 to 53), excellent discoloration inhibiting properties were obtained when the content of the component (C) was 1 to 7.5 wt%, and the HLB value (weighted average) of the component (C) was 8 to 15 (Examples 1, 4 to 6, 9 to 11, 13, 14 to 22, and 30 to 53), and more excellent discoloration inhibiting properties were obtained when the content of the component (C) was 1.5 to 7.5 wt%, and the HLB value (weighted average) of the component (C) was 11.5 to 15 (Examples 1, 4 to 6, 9 to 11, 14 to 17, 19 to 22, and 32 to 42).

Among the oil-in-water emulsion compositions of Examples 1 and 3 to 53 tested for the discoloration inhibiting properties, in the case of not containing a hydrocarbon and containing only a higher alcohol as the component (B) (Examples 26 to 29), excellent discoloration inhibiting properties were obtained when the component (D) contained solid polyalkylene glycol (Examples 28 and 29).

From the comparison between Example 86 and Example 87 and the comparison between Example 104 and Example 105, it was found that the adhesiveness was improved in the case of further containing a silicic acid compound (Examples 87 and 105).

### Test Example 2

The oil-in-water emulsion compositions shown in Table 11 were produced by the following procedure. First, the component (B) and the component (C) shown in Table 11 were mixed and dissolved by heating to prepare an oil phase composition. Separately, the component (A), the component (D), and light anhydrous silicic acid shown in Table 11 were mixed to prepare an aqueous phase composition. The aqueous phase composition was gradually added to the temperature-adjusted oil phase composition to refine particles (oil droplets) using a homomixer, and the mixture was cooled with stirring to obtain an oil-in-water emulsion composition. The obtained oil-in-water emulsion composition was evaluated for the separation inhibiting properties and the drug release properties in the same manner as in <1> and <7> of Test Example 1. The results are shown in Table 11. In Table 11, the results of Examples 10, 74, and 75 of Test Example 1 are also shown.

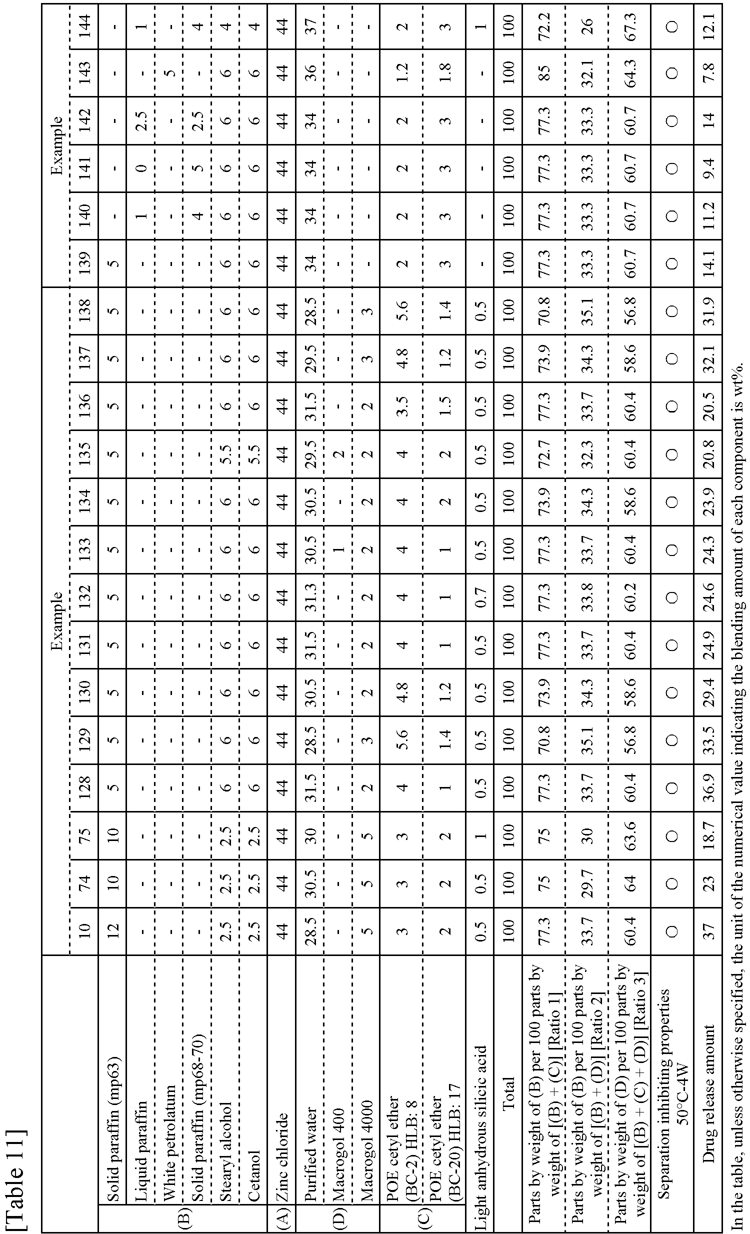

As shown in Examples 10, 74, 75, and 128 to 144, in the oil-in-water emulsion composition containing: (A) 30 wt% or more of zinc chloride; (B) an oily base selected from the group consisting of a hydrocarbon oil and a higher alcohol; (C) a non-ionic surfactant; and (D) an aqueous base selected from the group consisting of water and a polyalcohol, it has been confirmed that excellent separation inhibiting properties are obtained by satisfying the requirement that the content (ratio 1) of (B) with respect to 100 parts by weight of the total amount of the component (B) and the component (C) is 52 parts by weight or more, and the content (ratio 2) of (B) with respect to 100 parts by weight of the total amount of the component (B) and the component (D) is 11.7 parts by weight or more.

As shown in the comparison between Examples 10, 74, 75, and 128 to 138 and Examples 139 to 144, in the oil-in-water emulsion composition containing: (A) 30 wt% or more of zinc chloride; (B) an oily base selected from the group consisting of a hydrocarbon oil and a higher alcohol; (C) a non-ionic surfactant; and (D) an aqueous base selected from the group consisting of water and polyalcohols, it has been confirmed that the drug release properties are also improved when the component (D) is water and polyethylene glycol (Examples 10, 74, 75, and 128 to 138). As shown in the comparison between Examples 10, 74, and 128 to 138 and Example 75, it has also been confirmed that the drug release properties are further improved when the amount of light anhydrous silicic acid is 0.7 wt% or less (Examples 10, 74, and 128 to 138). This was also common with that, in comparison between Examples 10, 57 to 61, 64 to 65, 70 to 71, 73 to 74, 81, 83, 85, 87 to 90, 92 to 94, 97, 101, and 107 and Examples 75, 118, and 126 of Test Example 1, when the amount of light anhydrous silicic acid is 0.7 wt% or less (Examples 10, 57 to 61, 64 to 65, 70 to 71, 73 to 74, 81, 83, 85, 87 to 90, 92 to 94, 97, 101, and 107), the drug release properties are further improved.

## Claims

1. An oil-in-water emulsion composition comprising:
(A) 30 wt% or more of zinc chloride;
(B) an oily base selected from the group consisting of a hydrocarbon oil and a higher alcohol;
(C) a non-ionic surfactant; and
(D) an aqueous base selected from the group consisting of water and a polyalcohol,
wherein a content of (B) with respect to 100 parts by weight of the total amount of the component (B) and the component (C) is 52 parts by weight or more, and
a content of (B) with respect to 100 parts by weight of the total amount of the component (B) and the component (D) is 11.7 parts by weight or more.

2. An oil-in-water emulsion composition comprising:
(A) 30 wt% or more of zinc chloride;
(B) an oily base selected from the group consisting of a hydrocarbon oil and a higher alcohol;
(C) a non-ionic surfactant; and
(D) an aqueous base selected from the group consisting of water and polyalcohols,
wherein the component (D) is water and polyethylene glycol.

3. The oil-in-water emulsion composition according to claim 1 or 2, wherein a content of the component (D) with respect to 100 parts by weight of the total amount of the component (B), the component (C), and the component (D) is 42 parts by weight or more.

4. The oil-in-water emulsion composition according to claim 1 or 2, wherein a content of the component (C) is 12.7 wt% or less.

5. The oil-in-water emulsion composition according to claim 1 or 2, wherein the content of the component (C) is 1 to 7.5 wt%.

6. The oil-in-water emulsion composition according to claim 1 or 2, wherein the component (C) is one or more kinds of non-ionic surfactants, and
an HLB value of the component (C) represented by weighted-averaging an HLB of each non-ionic surfactant by the content is 8 to 17.

7. The oil-in-water emulsion composition according to claim 1, wherein the component (D) contains a polyalcohol, and the polyalcohol contains solid polyalkylene glycol.

8. The oil-in-water emulsion composition according to claim 1 or 2, wherein the component (B) contains both a hydrocarbon oil and a higher alcohol.

9. The oil-in-water emulsion composition according to claim 1 or 2, wherein the higher alcohol is a linear alcohol.

10. The oil-in-water emulsion composition according to claim 1 or 2, further comprising a silicic acid compound.

11. The oil-in-water emulsion composition according to claim 10, wherein a content of the silicic acid compound is 0.7 wt% or less.

12. The oil-in-water emulsion composition according to claim 1 or 2, wherein the component (C) is selected from the group consisting of polyoxyalkylene alkyl ether and polyoxyalkylene arachyl ether.

13. The oil-in-water emulsion composition according to claim 12, wherein the component (C) is two or more polyoxyalkylene alkyl ethers, and hydrophobic alkyl groups of the polyoxyalkylene alkyl ethers are the same as each other.

14. The oil-in-water emulsion composition according to claim 2, wherein the content of (B) with respect to 100 parts by weight of the total amount of the component (B) and the component (C) is 52 parts by weight or more, and
the content of (B) with respect to 100 parts by weight of the total amount of the component (B) and the component (D) is 11.7 parts by weight or more.
